# EUROPEAN PATENT SPECIFICATION

(11) **EP 0 492 171 B1**
(45) Date of publication and mention of the grant of the patent: **02.08.2000**
(21) Application number: 91120369.3
(22) Date of filing: 28.11.1991
(51) Int. Cl.: C07D 207/22, C07C 233/51, A01N 43/36

(54) **4-Chloro-1-(ethoxymethyl)-2-(p-chlorophenyl)-5-(trifluoromethyl)pyrrole-3-carbonitrile, and its use as insecticide, nematocide and acaricide**
4-Chlor-1-(ethoxymethyl)-2-(p-chlorophenyl)-5-(trifluoromethyl)pyrrol-3-carbonitril und dessen Verwendung als Insektizid, Nematozid und Acarizid
4-Chloro-1-(ethoxyméthyl)-2-(p-chlorophenyl)-5-(trifluorométhyl)pyrrole-3-carbonitrile, et son utilisation comme insecticide, nematocide et acaricide

(30) Priority: 26.12.1990 US 634288; 26.12.1990 US 634287
(43) Date of publication of application: 01.07.1992
(60) Divisional application: 97119172.1
(73) Proprietor: AMERICAN CYANAMID COMPANY, Wayne, NJ 07470-8426 (US)
(72) Inventor: Kameswaran, Venkataraman, Princeton Junction, New Jersey 08550 (US); Doehner, Robert Francis, Jr., East Windsor, New Jersey 08520 (US); Barton, Jerry Michael, East Windsor, New Jersey 08520 (US); Kuhn, David George, Newtown, Pennsylvania 18940 (US)
(74) Representative: Wächtershäuser, Günter, Prof. Dr.

(56) References cited:
- EP-A- 0 279 716
- EP-A- 0 281 707
- EP-A- 0 347 488
- EP-A- 0 358 047
- TETRAHEDRON LETTERS vol. 28, no. 52, 1987, GREAT BRITAIN pages 6597 - 6600; J.P.CELERIER ET AL.: 'Heterocyclization of primary amines with highly activated cyclopropanes:A new route to isoretronecanol'

## Description

EP- A 0347 488 discloses arylpyrrole insecticidal, acaricidal and nematocidal agents.

It is an object of this invention to provide an insecticidal, acaricidal and nematocidal agent which is 4-chloro-1-(ethoxymethyl)-2-(p-chlorophenyl)-5-(trifluoromethyl) pyrrole-3-carbonitrile and a method for its use to protect harvested and growing crops.

This compound of formula IV 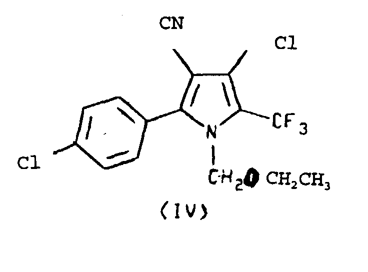 may be prepared by reacting a compound of formula (I) 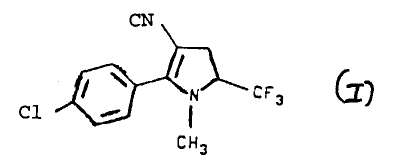 with at least 1 molar equivalent of a halogen, X₂, wherein X₂ is Br, Cl or I, preferably at an elevated temperature in the presence of a solvent to obtain a 2-aryl-1-methylpyrrole intermediate, reacting said 1-methyl-pyrrole intermediate with at least one additional molar equivalent of chlorine to form a 2-aryl-4-chloro-1-methylpyrrole intermediate, reacting said 4-chloro-1-methylpyrrole intermediate further with at least one molar equivalent of chlorine in the presence of a radical initiator to form a 2-aryl-4-chloro-1-(chloromethyl) pyrrole intermediate and reacting said 4-chloro-1-(chloromethyl) pyrrole intermediate with at least one molar equivalent of an alkali metal ethoxide to give the compound of formula IV.

The reactions are shown in Flow Diagram I.

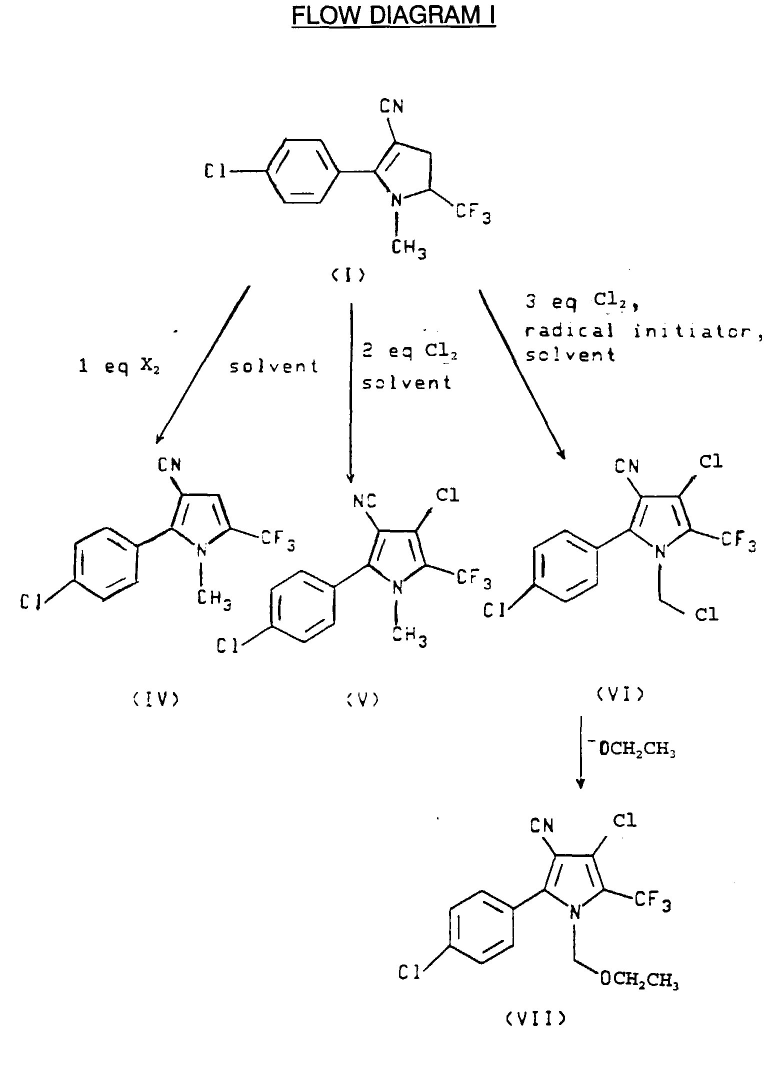

Advantageously, it has been found that the incremental addition of halogen improves the reaction yield and product purity. The process also improves the enviromental and human safety of the manufacture of compounds of formula IV by avoiding the use of certain undesirable reactants such as chloromethylethylether.

Further, the process allows the productive manufacture of 2-(p-chlorophenyl)-4-chloro-1-ethoxymethyl-5-(trifluoromethyl) pyrrole-3-carbonitrile, an effective insecticidal, acaricidal and nematocidal agent.

Solvents suitable for use in the process of the present invention include aprotic solvents, for example halogenated aromatic hydrocarbons such as chlorobenzene and o-dichlorobenzene and halogenated hydrocarbons such as methylene chloride, carbon tetrachloride, chloroform and, 1,2-dichloroethane. Preferred solvents include halogenated aromatic hydrocarbons such as chlorobenzene and halogenated hydrocarbons such as carbon tetrachloride. Radical initiators suitable for use are light, peroxides such as dibenzoyl peroxide and di-t-butylperoxide; azo compounds such as azobisisobutyronitrile. Among the preferred radical initiators are benzoyl peroxide and light. Alkali metal alkoxides suitable for use in the method are sodium ethoxide, and potassium methoxide.

In the process the reaction rate is increased with increased temperature so that elevated temperatures in the range of 55° to 210°C, preferably 70° to 120°C, allow the oxidation, ring halogenation and side chain halogenation to proceed at an efficient and effective rate without causing undue adverse side reactions.

It is now apparent that the process allows the operative manufacture of an insecticidal, acaricidal and nematocidal agent, 2-(p-chlorophenyl)-4-chloro-1-(ethoxymethyl)-5-(trifluoromethyl) pyrrole-3-carbonitrile. The pyrrole-3-carbonitrile is effective for controlling acarina, lepodoptera, colioptera and hemiptera. Among the agronomic pests that may be effectively controlled are potato beetle, cabbage looper, diamond back moth, tobacco budworm, corn earworm, beet armyworm, tobacco bollworm and the like. Crops which may be protected by the compound of the invention are lettuce, broccoli, corn, cabbage, cauliflower, tomato, cotton and so forth.

In practice, generally 10 ppm to 10,000 ppm, preferably 100 to 5,000 ppm of 2-(p-chlorophenyl)-4-chloro-1-(ethoxymethyl)-5-(trifluoromethyl) pyrrole-3-carbonitrile dispersed in water or other suitable liquid carrier, is effective when applied to the crops or the soil in which the crops are growing to protect the crops from attack by insects, acarina and nematodes. The compound is also effective for protecting horticultural plants such as turf grass from attack by pests such as grubs, chinch bugs and the like.

While the 4-chloro-1-(ethoxymethyl)pyrrole compound of the invention is effective for controlling insects, acarina and nematodes when employed alone, it may also be used in combination with other pesticidal compounds including other nematocides and acaricides. For example, the compound of this invention may be employed effectively in combination with other arylpyrroles, phosphates, carbamates, pyrethroids, formamidines, chlorinated hydrocarbons, and halobenzoylureas.

In order to facilitate a further understanding of the invention, the following examples are presented to illustrate more specific details thereof. The term NMR designates nuclear magnetic resonance and the term HPLC designates high pressure liquid chromatography. Unless otherwise noted, all parts are parts by weight.

### Reference example 1

### Preparation of 2-(p-Chlorophenyl) sarcosine

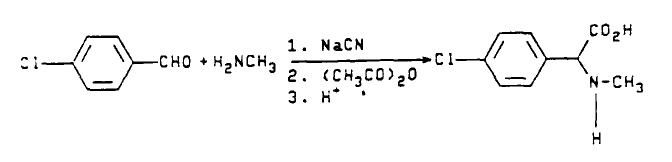

A mixture of p-chlorobenzaldehyde (153 g, 1.0 mol) in tetrahydrofuran is treated with a solution of methylamine hydrochloride (88 g, 1.3 mol) in water followed by an aqueous solution of sodium cyanide (53 g, 1.0 mol), stirred at room temperature for 16 hours and extracted with toluene. The organic extract is treated with 10 cc of pyridine followed by 50 cc of acetic anhydride (exotherm), stirred at ambient temperatures for 1/2 hour and concentrated in vacuo to give an oil residue. The residue is added to a 1:1 mixture of water and concentrated hydrochloric acid, heated at reflux temperature for 2 hours, cooled, diluted with water and neutralized to about pH 2 with 50% NaOH solution. The resultant solid precipitate is filtered and air-dried to give the title product as a white solid, 180 g (89.5% yield), mp 208 - 213°C.

### Reference example 2

### Preparation of 2-(p-Chlorophenyl)-N-(trifluoroacetyl) sarcosine

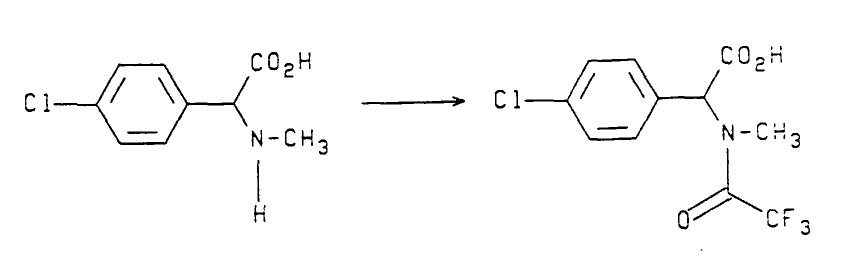

A mixture of 2-(p-chlorophenyl) sarcosine (27 g, 0.135 mol) in dry toluene is treated with 20 cc of trifluoroacetic anhydride, stirred for 1 hour and concentrated in vacuo to give a solid residue. The residue is re-evaporated several times with toluene to give the title product as a red solid, 38.7 g (97% yield), mp 117-118°C, identified by NMR spectroscopy.

### Reference example 3

### Preparation of 2-p-chlorophenyl-N-(trifluoroacetyl) glycine

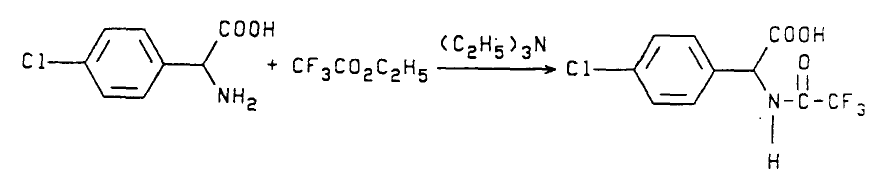

A mixture of 2-(p-chlorophenyl) glycine (37.1 g, 0.2 mol) in methanol is treated with triethylamine (20.2 g, 0.2 mol), stirred for 10 minutes, treated dropwise with ethyl trifluoroacetate (35.5 g, 0.25 mol), stirred for 4 days, diluted with methanol and treated with Dowex 50x8-100 ion exchange resin. The reaction mixture is stirred for 10 minutes and filtered. The filter cake is washed with methanol. The filtrates are combined and concentrated in vacuo to afford a yellow solid which is recrystallized from 1,2-dichloroethane to give the title compound as white crystals, 26.4 g (46.8% yield), mp 170-172°C.

### Reference example 4

### Preparation of 2-(p-Chlorophenyl)-1-methyl-5-(tri-fluoromethyl)-2-pyrroline-3-carbonitrile

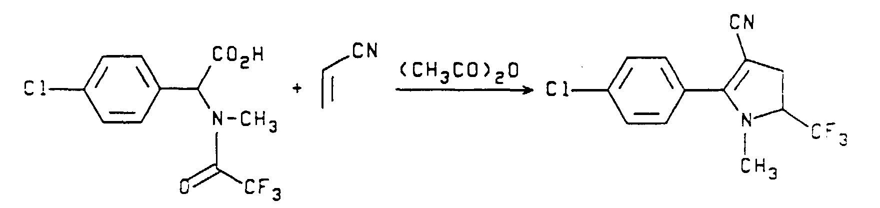

A solution of 2-(p-chlorophenyl)-N-trifluoromethyl)sarcosine (7.4 g, 0.02 mol) in acetonitrile is treated with acetic anhydride (5.1 g, 0.05 mol), acrylonitrile (1.6 g, 0.03 mol) and 10 drops of triethylamine, heated at reflux temperature for 5 1/2 hours, cooled and concentrated in vacuo to a red oil residue. The residue is filtered through silicagel using 9:1 hexanes/ethyl acetate followed by mixtures of methylene chloride and ethyl acetate. The combined filtrates are concentrated in vacuo to give the title product as a red solid, 6.1g (85% yield), identified by NMR and mass spectral analyses. A portion of the solid is recrystallized from methylene chloride to give light yellow needles, mp 158-160°C.

### Reference example 5

### Preparation of 4-Chloro-1-chloromethyl-2-(p-chlorophenyl)-5-(trifluoromethyl) pyrrole-3-carbonitrile

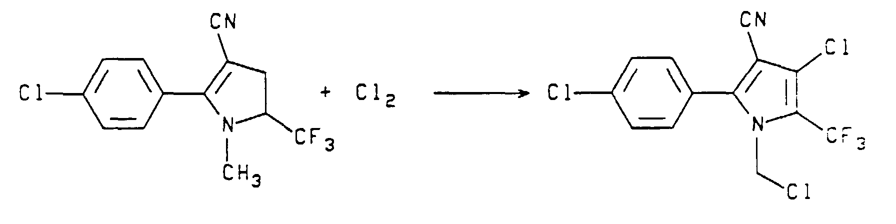

To a solution of 2-(p-chlorophenyl)-1-methyl-5-(trifluoromethyl)-2-pyrroline-3-carbonitrile (2.85 g, 0.01 mol) in o-dichlorobenzene is added chlorine (0.8 g, 0.01 mol); the resulting solution is stirred at room temperature for 1 hour, heated slowly to 90° C over a 2 hour period, cooled to 50°C, treated with additional chlorine (0.8 g, 0.11 mol), heated at 110° C for 24 hours, cooled to room temperature, treated with additional chlorine and concomitant additions of small portions of benzoyl peroxide and heated at 110°C for a 4 hour period or until reaction is complete by chromatographic analysis. The resultant solution is cooled, washed with sodium metabisulfite solution and concentrated in vacuo to give a residue which is crystallized by the addition of heptane to afford the title product as a white solid, mp 107-108° C.

### Reference example 6

### Preparation of 2-(p)-Chlorophenyl)-1-methyl-5-(trifluoromethyl) pyrrole-3-carbonitrile

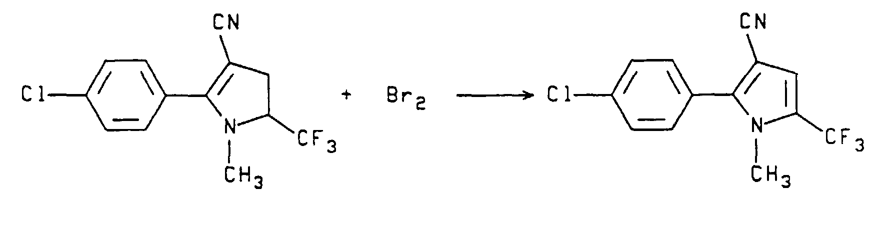

A mixture of 2-(p-chlorophenyl)-1-methyl-5-(trifluoromethyl)-2-pyrroline-3-carbonitrile (2.87 g, 0.01 mol) in chlorobenzene is treated with a solution of bromine (1.76 g, 0.011 mol) in chlorobenzene and heated at 100° C for 4-5 hours (until the reaction is complete by HPLC analysis). The reaction mixture is cooled to room temperature, diluted with water and extracted with ethyl acetate. The organic extracts are combined, washed sequentially with water, sodium metabisulfite and water, dried over MgSO₄ and concentrated in vacuo to give a pale yellow solid residue. The solid is recrystallized from heptane/ethyl acetate to give the title product as pale yellow crystals, 2.4 g, (84.2% yield), mp 129.5-130°C.

### Reference example 7

### Preparation of 4-Chloro-1-(chloromethyl)-2-(p-chlorophenyl)-5-(trifluoromethyl) pyrrole-3-carbonitrile

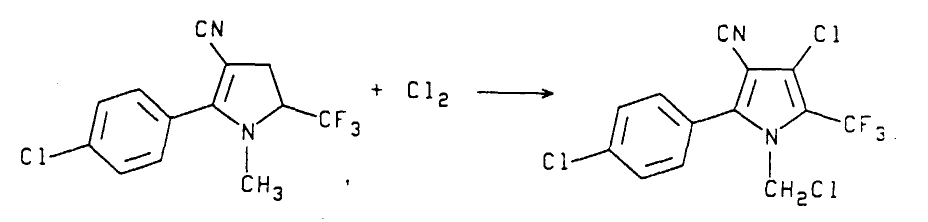

To a solution of 2-(p-chlorophenyl)-1-methyl-5-(trifluoromethyl)-2-pyrroline-3-carbonitrile (2.85 g, 0.01 mol) in chlorobenzene is added chlorine (0.8 g, 0.011 mol), stirred at room temperature for 1 hour, heated at 90°C for 2 hours, cooled to room temperature, treated with additional chlorine (1.1 g, 0.015 mol), heated at 110°C for 24 hours, cooled to room temperature, treated further with additional chlorine (1.4 g, 0.02 mol) and a catalytic amount of benzoyl peroxide and heated at 110°C until reaction is complete by HPLC analysis. The mixture is cooled to room temperature, washed with aqueous sodium metabisulfite, dried (MgSO₄), and concentrated in vacuo to give a residue. The residue is recrystallized from heptane to give the title product as a white crystalline solid, mp 107-108° C.

### Example 1

### Preparation of 4-Chloro-2-(p-chlorophenyl)-1-(ethoxymethyl)-5-(trifluoromethyl) pyrrole-3-carbonitrile

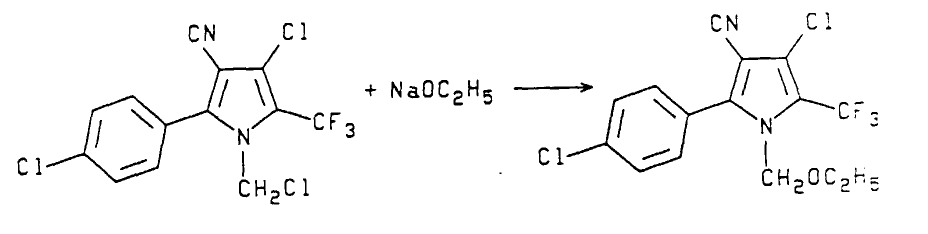

A solution of 4-chloro-1-(chloromethyl)-2-(p-chlorophenyl)-5-(trifluoromethyl) pyrrole-3-carbonitrile (2.6 g, 0.0074 mol) in tetrahydrofuran is treated with sodium ethoxide as a 21% wt/wt solution in denatured ethanol (3.6 mL, 0.0096 mol), stirred at room temperature for 1 hour, treated with an additional 2-3 drops of the sodium ethoxide solution, heated at reflux temperature for 1 hour, cooled and poured in water. The resultant precipitate is filtered, dried and recrystallized from isopropanol to afford the title product as a white solid, 1.6 g (60% yield), mp 104.0- 104.5°C.

### Example 2

### Insecticide and Acaricide Evaluation

All tests are performed using technical material. All concentrations reported herein are in terms of active ingredient. All tests are kept at 27°C.

### Spodoptera eridania, 3rd instar larvae, southern armyworm

A Sieva lima bean leaf expanded to 7-8 cm in length is dipped in the test suspension with agitation for 3 seconds and placed in a hood to dry. The leaf is then placed in a 100x10 mm petri dish containing a damp filter paper on the bottom and ten 3rd instar caterpillars. The dish is maintained for 5 days before observations are made of mortality, reduced feeding, or any interference with normal moulting.

### Spodoptera eridania, 7 day residual

The plants treated in the above test are maintained under high intensity lamps in the greenhouse for 7 days. These lamps duplicate the effects of a bright sunny day in June in New Jersey and are kept on for 14 hour day length. After 7 days, the foliage is sampled and evaluated as described above.

### Tetranychus urticae (P-resistant strain), 2-spotted spider mite

Sieva lima bean plants with primary leaves expanded to 7-8 cm are selected, cut back to one plant per pot and infested with mites to obtain about 100 mites per leaf. The mite-infested plants are dipped in the test formulation for 3 seconds with agitation and set in the hood to dry. Plants are kept for 2 days before estimates of adult kill are made using the first leaf. The second leaf is kept on the plant for another 5 days before observations are made of the kill of eggs and/or newly emerged nymphs.

### Diabrotic undecimpunctata howardi, 3rd instar southern corn rootworm

One cc of fine talc is placed in a 30 mL wide-mouth screw-top glass jar. One mL of the appropriate acetone suspension is pipetted onto the talc so as to provide 1.25 and 0.25 mg of active ingredient per jar. The jars are set under a gentle air flow until the acetone is evaporated. The dried talc is loosened, 1 cc of millet seed is added to serve as food for the insects and 25 mL of moist soil is added to each jar. The jar is capped and the contents thoroughly mixed on a Vortex Mixer. Following this, ten 3rd instar rootworms are added to each jar and the jars are loosely capped to allow air exchange for the larvae. The treatments are held for 6 days before mortality counts are made. Missing larvae are presumed dead, since they decompose rapidly and can not be found. The concentrations used in this test correspond approximately to 50 and 10 kg/ha, respectively.

### Aphis fabae, mixed instar, bean aphid

Pots containing single nasturtium plants (Tropaeolum sp) about 5 cm tall are infested with about 100-200 aphids one day before the test. Each pot is sprayed with the test formulation for 2 revolutions of a 4 rpm turntable in a hood, using a #154 DeVilDiss atomizer. The spray tip is held about 15 cm from the plant and the spray directed so as to give complete coverage of the plants and the aphids. The sprayed pots are set on their sides on white enamel trays and held for 2 days, following which mortality estimates are made.

### Empoasca abrupta, adults, western potato leafhopper

A Sieva lima bean leaf about 5 cm long is dipped in the test formulation for 3 seconds with agitation and placed in a hood to dry. The leaf is placed in a 100x10 mm petri dish containing a moist filter paper on the bottom. About 10 adjult leafhoppers are added to each dish and the treatments are kept for 3 days before mortality counts are made.

### Heliothis virescens, 3rd instar tobacco budworm

Cotton cotyledons are dipped in the test formulation and allowed to dry in a hood. When dry, each is cut into quarters and ten sections placed individually in 30 mL plastic medicine cups containing a 5-7 mm long piece of damp dental wick. One third-instar caterpillar is added to each cup and a cardboard lid placed on the cup. Treatments are maintained for 3 days before mortality counts and estimates of reduction in feeding damage are made.

### Heliothis virescens, egg, tobacco budworm

A young cotton leaf about 6-7 cm long is dipped in the test suspension with agitation for 3 seconds. Eggs are collected on cheesecloth which is cut into 10-20 mm squares containing about 50-100 eggs (6-30 hours old). A square of cheesecloth with eggs is also dipped in the test suspension and placed on the treated leaf. The combination is dried and placed in a cup (240 mL, 6 cm tall, top diameter 9.5 cm, bottom diameter 8 cm), into which a 5 cm length of damp dental wick has been placed. A clear plastic lid is placed on the cup and the treatments are held for 3 days before mortality counts are made.

### Blattela germanica, bait test, adult male German cockroach

A 0.1% bait is prepared by pipetting a 1 mL of a 1000 ppm solution of the test ompound in acetone onto 1 gram of cornmeal in a 30 mL wide-mouth bottle. The bait is dried by passing a gentle stream of air into the bottle. The bait is placed in a 1 pint wide-mouth Mason jar and ten adult male cockroaches are added. A screen lid is placed on the jar and a small piece of cotton soaked in 10% honey is put on the top of the screen lid. Mortality counts are made after 3 days.

### Blattela germanica, residue test, adult male German cockroach

One mL of a 1000 ppm acetone solution of the test material is pipetted slowly over the bottom of a 150 x 15 mm petri dish so as to give as uniform coverage as possible. After the deposit has dried, 10 adult male cockroaches are placed in each dish and the lid is added. Mortality counts are made after 3 days.

| Rating Scale: | |
|---|---|
| 0 = no effect | 5 = 56 - 65% kill |
| 1 = 10 - 25% kill | 6 = 66 - 75% kill |
| 2 = 26 - 35% kill | 7 = 76 - 85% kill |
| 3 = 36 - 45% kill | 8 = 86 - 99% kill |
| 4 = 46 - 55% kill | 9 = 100% kill |
| | R - reduced feeding |

The data obtained for the above-described evaluations are reported in Table I.

**TABLE I**

| **Insecticide and Acaricide Evaluation of 2- (p-chlorophenyl)-4-chloro-1-(ethoxymethyl)-5-(trifluoromethyl) pyrrole-3-carbonitrile** | | | | | |
|---|---|---|---|---|---|
| Test | Evaluation | | | | |
| | 1,000 ppm | 100 ppm | 10 ppm | 1 ppm | 0.1 ppm |
| Southern Armyworm | | | | | |
| (3rd instar larvae) | 9 | 9 | 9 | 0 | - |
| (7 day residual) | 9 | 9 | 6 | - | - |
| 2-Spotted Spider Mite | - | - | 9 | 0 | - |
| Bean Aphid | - | 9 | 4 | 0 | - |
| Leafhopper | - | 9 | 9 | 9 | - |

| Tobacco Budworm | | | | | |
|---|---|---|---|---|---|
| (Eggs) | 7 | 0 | - | - | - |
| (3rd instar) | 9 | 9 | 9 | - | - |

| German Cockroach | | | | | |
|---|---|---|---|---|---|
| (Bait) | 0 | - | - | - | - |
| (Residue) | 9 | 0 | - | - | - |

| | 50 kg/ha | | 10 kg/ha | 1 kg/ha | |
|---|---|---|---|---|---|
| Southern Corn Rootworm | 9 | | 7 | 0 | |

## Claims (Claims for the following Contracting State(s): AT, BE, CH, LI, DE, DK, FR, GB, IT, LU, NL, SE)

1. A compound which is 4-chloro-1-(ethoxymethyl)-2-(p-chlorophenyl)-5-(trifluoromethyl) pyrrole-3-carbonitrile.

2. A method for protecting growing plants from attack by insects, nematodes and acarina which is characterized by applying to the foliage of said plants or to the soil or water in which said plants are growing, an insecticidally, nematocidally or acaricidally effective amount of the compound according to claim 1.

3. A method for controlling insects, nematodes and acarina which is characterized by contacting said insects, nematodes and acarince, their breeding grounds, food supply or habitat with an insecticidally, nematocidally or acaricidally effective amount of the compound according to claim 1.

4. A composition for protecting growing plants from attack by insects, nematodes and acarina characterized by an agronomically acceptable carrier containing an insecticidally, nematocidally, or acaricidally effective amount of the compound according to claim 1.

5. A composition for controlling insects, nematodes and acarina characterized by an agronomically acceptable carrier containing an insecticidally, nematocidally or acaricidally effective amount of the compound according to claim 1.

## Claims (Claims for the following Contracting State(s): ES)

1. A method for protecting growing plants from attack by insects, nematodes and acarina which is characterized by applying to the foliage of said plants or to the soil or water in which said plants are growing, an insecticidally, nematocidally or acaricidally effective amount of 4-chloro-1-(ethoxymethyl)-2-(p-chlorophenyl)-5-(trifluoromethyl) pyrrole-3-carbonitrile.

2. A method for controlling insects, nematodes and acarina which is characterized by contacting said insects, nematodes and acarince, their breeding grounds, food supply or habitat with an insecticidally, nematocidally or acaricidally effective amount of the compound according to claim 1.

3. Use of a compound defined in claim 1 in a composition for protecting growing plants from attack by insects, nematodes and acarina characterized by an agronomically acceptable carrier containing an insecticidally, nematocidally, or acaricidally effective amount of the compound according to claim 1.

4. Use of a compound defined in claim 1 in a composition for controlling insects, nematodes and acarina characterized by an agronomically acceptable carrier containing an insecticidally, nematocidally or acaricidally effective amount of the compound according to claim 1.

## Claims (Claims for the following Contracting State(s): GR)

1. A method for protecting growing plants from attack by insects, nematodes and acarina which is characterized by applying to the foliage of said plants or to the soil or water in which said plants are growing, an insecticidally, nematocidally or acaricidally effective amount of 4-chloro-1-(ethoxymethyl)-2-(p-chlorophenyl)-5-(trifluoromethyl) pyrrole-3-carbonitrile.

2. A method for controlling insects, nematodes and acarina which is characterized by contacting said insects, nematodes and acarince, their breeding grounds, food supply or habitat with an insecticidally, nematocidally or acaricidally effective amount of the compound according to claim 1.

3. A composition for protecting growing plants from attack by insects, nematodes and acarina characterized by an agronomically acceptable carrier containing an insecticidally, nematocidally, or acaricidally effective amount of the compound according to claim 1.

4. A composition for controlling insects, nematodes and acarina characterized by an agronomically acceptable carrier containing an insecticidally, nematocidally or acaricidally effective amount of the compound according to claim 1.

## Patentansprüche (Patentansprüche für folgende(n) Vertragsstaat(en): AT, BE, CH, LI, DE, DK, FR, GB, IT, LU, NL, SE)

1. Verbindung, die 4-Chlor-1-(ethoxymethyl)-2-(p-chlorphenyl)-5-(trifluormethyl)pyrrol-3-carbonitril ist.

2. Verfahren zum Schutz von wachsenden Pflanzen vor dem Angriff von Insekten, Nematoden und Acarina, das dadurch gekennzeichnet ist, daß man auf die Blätter der Pflanzen oder auf die Erde oder in das Wasser, worin die Pflanzen wachsen, eine insektizid, nematozid oder akarizid wirksame Menge der Verbindung nach Anspruch 1 auf- bzw. einbringt.

3. Verfahren zur Kontrolle von Insekten, Nematoden und Acarina, das dadurch gekennzeichnet ist, daß man die Insekten, Nematoden und Acarina, ihre Brutstätten, ihren Futtervorrat oder Lebensraum mit einer insektizid, nematozid oder akarizid wirksamen Menge der Verbindung nach Anspruch 1 in Kontakt bringt.

4. Zusammensetzung zum Schutz von wachsenden Pflanzen vor dem Angriff durch Insekten, Nematoden und Acarina, gekennzeichnet durch einen landwirtschaftlich annehmbaren Träger, der eine insektizid, nematozid oder akarizid wirksame Menge der Verbindung nach Anspruch 1 enthält.

5. Zusammensetzung zur Kontrolle von Insekten, Nematoden und Acarina, gekennzeichnet durch einen landwirtschaftlich annehmbaren Träger, der eine insektizid, nematozid oder akarizid wirksame Menge der Verbindung nach Anspruch 1 enthält.

## Patentansprüche (Patentansprüche für folgende(n) Vertragsstaat(en): ES)

1. Verfahren zum Schutz von wachsenden Pflanzen vor einem Angriff von Insekten, Nematoden und Acarina, das dadurch gekennzeichnet ist, daß man auf die Blätter der Pflanzen oder auf die Erde oder in das Wasser, worin die Pflanzen wachsen, eine insektizid, nematozid oder akarizid wirksame Menge von 4-Chlor-1-(ethoxymethyl)-2-(p-chlorphenyl)-5-(trifluormethyl)pyrrol-3-carbonitril auf- bzw. einbringt.

2. Verfahren zur Kontrolle von Insekten, Nematoden und Acarina, das dadurch gekennzeichnet ist, daß man Insekten, Nematoden und Acarina, ihre Brutstätten, ihren Futtervorrat oder Lebensraum mit einer insektizid, nematozid oder akarizid wirksamen Menge der Verbindung nach Anspruch 1 in Kontakt bringt.

3. Verwendung einer Verbindung, die in Anspruch 1 definiert ist, in einer Zusammensetzung zum Schutz von wachsenden Pflanzen vor einem Angriff durch Insekten, Nematoden und Acarina, gekennzeichnet durch einen landwirtschaftlich annehmbaren Träger, der eine insektizid, nematozid oder akarizid wirksame Menge der Verbindung nach Anspruch 1 enthält.

4. Verwendung einer Verbindung, die in Anspruch 1 definiert ist, in einer Zusammensetzung zur Kontrolle von Insekten, Nematoden und Acarina, gekennzeichnet durch einen landwirtschaftlich annehmbaren Träger, der eine insektizid, nematozid oder akarizid wirksame Menge der Verbindung nach Anspruch 1 enthält.

## Patentansprüche (Patentansprüche für folgende(n) Vertragsstaat(en): GR)

1. Verfahren zum Schutz von wachsenden Pflanzen vor dem Angriff von Insekten, Nematoden und Acarina, das dadurch gekennzeichnet ist, daß man auf die Blätter der Pflanzen oder auf die Erde oder in das Wasser, worin die Pflanzen wachsen, eine insektizid, nematozid oder akarizid wirksame Menge von 4-Chlor-1-(ethoxymethyl)-2-(p-chlorphenyl)-5-(trifluormethyl)pyrrol-3-carbonitril auf- bzw.einbringt.

2. Verfahren zur Kontrolle von Insekten, Nematoden und Acarina, das dadurch gekennzeichnet ist, daß man die Insekten, Nematoden und Acarina, ihre Brutstätten, ihren Futtervorrat oder Lebensraum mit einer insektizid, nematozid oder akarizid wirksamen Menge der Verbindung nach Anspruch 1 in Kontakt bringt.

3. Zusammensetzung zum Schutz von wachsenden Pflanzen vor dem Angriff durch Insekten, Nematoden und Acarina, gekennzeichnet durch einen landwirtschaftlich annehmbaren Träger, der eine insektizid, nematozid oder akarizid wirksame Menge der Verbindung nach Anspruch 1 enthält.

4. Zusammensetzung zur Kontrolle von Insekten, Nematoden und Acarina, gekennzeichnet durch einen landwirtschaftlich annehmbaren Träger, der eine insektizid, nematozid oder akarizid wirksame Menge der Verbindung nach Anspruch 1 enthält.

## Revendications (Revendications pour l'(les) Etat(s) contractant(s) suivant(s): AT, BE, CH, LI, DE, DK, FR, GB, IT, LU, NL, SE)

1. Composé qui est le 4-chloro-1-(éthoxyméthyl)-2-(*p*-chlorophényl)-5-(trifluorométhyl)pyrrole-3-carbonitrile.

2. Procédé pour protéger des plantes en çroissance contre une attaque par des insectes, nématodes et acariens, qui est caractérisé par l'application au feuillage desdites plantes ou à la terre ou à l'eau dans laquelle ces plantes croissent, d'une quantité à effet insecticide, nématicide ou acaricide du composé selon la revendication 1.

3. Procédé pour combattre des insectes, nématodes et acariens, qui est caractérisé par la mise en contact desdits insectes, nématodes et acariens, de leurs lieux de reproduction, de leur source de nourriture ou de leur habitat, avec une quantité à effet insecticide, nématicide ou acaricide du composé selon la revendication 1.

4. Composition destinée à protéger des plantes en croissance contre une attaque par des insectes, nématodes et acariens, caractérisée par un support acceptable en usage agronomique contenant une quantité à effet insecticide, nématicide ou acaricide du composé selon la revendication 1.

5. Composition destinée à combattre des insectes, nématodes et acariens, caractérisée par un support acceptable en usage agronomique contenant une quantité à effet insecticide, nématicide ou acaricide du composé selon la revendication 1.

## Revendications (Revendications pour l'(les) Etat(s) contractant(s) suivant(s): ES)

1. Procédé pour la protection des plantes en croissance contre une attaque par des insectes, nématodes et acariens, caractérisé en ce qu'il consiste à appliquer sur le feuillage desdites plantes ou à la terre ou à l'eau dans laquelle ces plantes croissent une quantité à effet insecticide, nématicide ou acaricide, de 4-chloro-1-(éthoxyméthyl)-2-(p-chlorophényl)-5-(trifluorométhyl)pyrrole-3-carbonitrile.

2. Procédé destiné à combattre des insectes, nématodes et acariens, caractérisé en ce qu'il consiste à mettre en contact lesdits insectes nématodes et acariens, leurs lieux de reproduction, leur source de nourriture ou leur habitat avec une quantité à effet insecticide, nématicide ou acaricide, du composé selon la revendication 1.

3. Utilisation d'un composé tel que défini à la revendication 1 dans une composition destinée à protéger des plantes en croissance contre une attaque par des insectes, nématodes et acariens, caractérisée par un support acceptable du point de vue agronomique contenant une quantité à effet insecticide, nématicide ou acaricide, du composé selon la revendication 1.

4. Utilisation d'un composé tel que défini à la revendication 1 dans une composition destinée à combattre des insectes, nématodes et acariens, caractérisée par un support acceptable sur le plan agronomique contenant une quantité à effet insecticide, nématicide ou acaricide, du composé selon la revendication 1.

## Revendications (Revendications pour l'(les) Etat(s) contractant(s) suivant(s): GR)

1. Procédé destiné à protéger des plantes en croissance contre une attaque par des insectes, nématodes et acariens, caractérisé en ce qu'il consiste à appliquer sur le feuillage desdites plantes ou à la terre ou à l'eau dans laquelle ces plantes croissent une quantité à effet insecticide, nématicide ou acaricide, de 4-chloro-1-(éthoxyméthyl)-2-(p-chlorophényl)-5-(trifluorométhyl)pyrrole-3-carbonitrile.

2. Procédé destiné à combattre des insectes, nématodes et acariens, caractérisé en ce qu'il consiste à mettre en contact lesdits insectes, nématodes et acariens, leurs lieux de reproduction, leur source de nourriture ou leur habitat avec une quantité à effet insecticide, nématicide ou acaricide, du composé selon la revendication 1.

3. Composition destinée à protéger des plantes en croissance contre une attaque par des insectes, nématodes et acariens, caractérisée par un support acceptable sur le plan agronomique contenant une quantité à effet insecticide, nématicide ou acaricide, du composé selon la revendication 1.

4. Composition destinée à combattre des insectes, nématodes et acariens, caractérisée par un support acceptable sur le plan agronomique contenant une quantité à effet insecticide, nématicide ou acaricide, du composé selon la revendication 1.
